# FASCICULE DE BREVET EUROPEEN

(11) **EP 3 145 909 B1**
(45) Date de publication et mention de la délivrance du brevet: **01.11.2023**
(21) Numéro de dépôt: 15725548.0
(22) Date de dépôt: 19.05.2015
(51) Int. Cl.: C07C 291/02, C07C 291/06, C08F 8/30, C08K 5/32, C08L 9/00, B60C 1/00, C08C 19/22, C08K 3/36

(54) **COMPOSE 1,3-DIPOLAIRE PORTANT UNE FONCTION ESTER D'ACIDE CARBOXYLIQUE ET COMPOSITION DE CAOUTCHOUC LE CONTENANT**
1,3-DIPOLARE VERBINDUNG MIT EINER ESTERFUNKTIONEN VON CARBONSÄURE UND KAUTSCHUKZUSAMMENSETZUNG DAMIT
1,3-DIPOLAR COMPOUND COMPRISING AN ESTER FUNCTION OF CARBOXYLIC ACID AND RUBBER COMPOSITION CONTAINING SAME

(30) Priorité: 23.05.2014 FR 1454655
(43) Date de publication de la demande: 29.03.2017
(73) Titulaire: Compagnie Générale des Etablissements Michelin, 63000 Clermont-Ferrand (FR)
(72) Inventeur: IVANOV, Sergey Nicolaevich, 63040 Clermont-Ferrand Cedex 9 (FR); SALIT, Anne-Frédérique, 63040 Clermont-Ferrand Cedex 9 (FR)
(74) Mandataire: Gandon-Pain, Sylvie
(86) Numéro de dépôt international: PCT/EP2015/060926
(87) Numéro de publication internationale: WO 2015/177104

(56) Documents cités:
- EP-A2- 1 802 593
- WO-A1-84/03883
- WO-A1-2012/095425
- GB-A- 1 174 922
- JP-A- 2008 163 232
- US-A1- 2006 084 730
- US-A1- 2012 046 418
- T. Polohski ET AL: "Nitrones as Intermediates in the Synthesis of N-Hydroxyamino Acid Esters", J. Org. Chem Vol. 41, No. 12,1976, 1 janvier 1976 (1976-01-01), pages 2092-2095, XP055161933, Extrait de l'Internet: URL:http://pubs.acs.org/doi/pdf/10.1021/jo 00874a004 [extrait le 2015-01-14]
- A. P. YAKUBOV ET AL: "Synthesis of stable functionally substituted nitrile oxides of the aromatic series", BULLETIN OF THE ACADEMY OF SCIENCES OF THE USSR; DIVISION OF CHEMICAL SCIENCES, vol. 40, no. 5, 1 mai 1991 (1991-05-01), pages 1078-1080, XP055161681, ISSN: 0568-5230, DOI: 10.1007/BF00961380
- N. F. BONDAR' ET AL: "Synthesis of 9,11-Ethano-13,15-isoxazolinoprostanoids, PGH Analogs", RUSSIAN JOURNAL OF ORGANIC CHEMISTRY, vol. 39, no. 8, 1 août 2003 (2003-08-01), pages 1089-1094, XP055161043, ISSN: 1070-4280, DOI: 10.1023/B:RUJO.0000010175.39145.9a
- JIH RU HWU ET AL: "SELECTIVITY OF THE BULKY PROTON-CONTAINING REAGENT N-METHYL-N,O-BIS(TRIMETHYLSILYL)HYDROXYLAM INE IN THE FORMATION OF NITRONES", JOURNAL OF ORGANOMETALLIC CHEMISTRY, ELSEVIER-SEQUOIA S.A. LAUSANNE, CH, vol. 399, no. 3, 11 décembre 1990 (1990-12-11), pages C13-C17, XP000169095, ISSN: 0022-328X, DOI: 10.1016/0022-328X(90)85501-O
- DANIELE SIMONI ET AL: "Heterocycle-Containing Retinoids. Discovery of a Novel Isoxazole Arotinoid Possessing Potent Apoptotic Activity in Multidrug and Drug-Induced Apoptosis-Resistant Cells", JOURNAL OF MEDICINAL CHEMISTRY, vol. 44, no. 14, 1 juillet 2001 (2001-07-01), pages 2308-2318, XP055161757, ISSN: 0022-2623, DOI: 10.1021/jm0010320
- Dennis Liotta ET AL: "Reactions of N-Aryl Nitrogen Oxides. 1. Selective Ortho Chlorination in the Reactions of Aryl Nitrones and Amine Oxides with Thionyl Chloride or Phosgene", J. Org. Chem, vol. 39, N 18, 1 janvier 1974 (1974-01-01), pages 2718-2722, XP055161892, Extrait de l'Internet: URL:http://pubs.acs.org/doi/pdf/10.1021/jo 00932a011 [extrait le 2015-01-14]
- CRAIG S. MCKAY ET AL: "Nitrones as dipoles for rapid strain-promoted 1,3-dipolar cycloadditions with cyclooctynes", CHEMICAL COMMUNICATIONS, vol. 46, no. 6, 1 janvier 2010 (2010-01-01), page 931, XP055105232, ISSN: 1359-7345, DOI: 10.1039/b921630h
- NAKAMA K ET AL: "Enantioselective conjugate additions of aldoximes to 3-crotonoyl-2-oxazolidinone and 1-crotonoyl-3-phenyl-2-imidazolidinone catalyzed by the aqua complex between R,R-DBFOX/Ph and zinc(II) perchlorate", TETRAHEDRON LETTERS, PERGAMON, GB, vol. 43, no. 5, 28 janvier 2002 (2002-01-28), pages 829-832, XP004332818, ISSN: 0040-4039, DOI: 10.1016/S0040-4039(01)02247-X
- BRYCE C. OXENRIDER ET AL: "Reactions of 2,2-dialkoxy ketone oximes with chlorine and bromine. Halogenation vs. Beckmann fragmentation", THE JOURNAL OF ORGANIC CHEMISTRY, vol. 47, no. 13, 1 juin 1982 (1982-06-01), pages 2629-2633, XP055161046, ISSN: 0022-3263, DOI: 10.1021/jo00134a023
- Beatrice Anichini ET AL: "Versatile Synthetic Approaches Towards Aza-analogues of Illudin and Ptaquilosin Sesquiterpenes", Synlett, 1 janvier 1997 (1997-01-01), pages 25-26, XP055161668, Extrait de l'Internet: URL:https://www.thieme-connect.de/products /ejournals/pdf/10.1055/s-1997-684.pdf [extrait le 2015-01-13]
- YASUHITO KOYAMA ET AL: "Cascade functionalization of unsaturated bond-containing polymers using ambident agents possessing both nitrile N-oxide and electrophilic functions", CHEMICAL COMMUNICATIONS, vol. 48, no. 83, 1 janvier 2012 (2012-01-01), page 10304, XP055161672, ISSN: 1359-7345, DOI: 10.1039/c2cc35158g
- T. Polohski ET AL: "Nitrones as Intermediates in the Synthesis of N-Hydroxyamino Acid Esters", J. Org. Chem Vol. 41, No. 12,1976, 1 January 1976 (1976-01-01), pages 2092-2095, XP055161933, Retrieved from the Internet: URL:http://pubs.acs.org/doi/pdf/10.1021/jo 00874a004 [retrieved on 2015-01-14]
- Morio Yonekawa ET AL: "Intramolecular 1,3-Dipolar Cycloaddition of Nitrile N -Oxide Accompanied by Dearomatization", ORGANIC LETTERS, vol. 14, no. 4, 17 February 2012 (2012-02-17), pages 1164-1167, XP055685415, ISSN: 1523-7060, DOI: 10.1021/ol300125s
- Yasuhito Koyama ET AL: "Cascade functionalization of unsaturated bond-containing polymers using ambident agents possessing both nitrile N-oxide and electrophilic functions", Chemical Communications, vol. 48, no. 83, 1 January 2012 (2012-01-01), page 10304, XP055461889, UK ISSN: 1359-7345, DOI: 10.1039/c2cc35158g

## Description

Le domaine de la présente invention est celui des compositions de caoutchouc diénique renforcées par une charge inorganique et utilisables notamment pour la fabrication de pneumatiques pour véhicules. Elle se rapporte plus particulièrement aux bandes de roulement des pneumatiques à faible résistance au roulement.

Idéalement, une bande de roulement pour pneumatique doit être la moins hystérétique possible pour minimiser sa contribution à la résistance au roulement du pneumatique. En parallèle une bande de roulement doit être suffisamment résistante à l'usure, ce qui implique que la composition de caoutchouc qui la constitue doit être suffisamment cohésive. Par ailleurs une composition de caoutchouc pour bande de roulement de pneumatique doit présenter un certain niveau de rigidité à cuit pour pouvoir assurer un bon comportement routier au pneumatique. Une composition de caoutchouc avec un bon niveau de cohésion ou un bon niveau de rigidité peut être obtenue en introduisant dans la composition de caoutchouc des taux relativement élevés de charges renforçantes, préférentiellement fines, ou en réduisant la quantité de plastifiant dans la composition de caoutchouc. Ces méthodes ont pour effet d'augmenter l'hystérèse de la composition de caoutchouc ou de dégrader la performance industrielle dans les étapes de fabrication de la composition de caoutchouc et de la bande de roulement, notamment en raison d'une élévation de la viscosité à l'état cru de la composition de caoutchouc.

Il est donc une préoccupation constante de trouver des compositions de caoutchouc qui offre un bon compromis entre cohésion, rigidité et hystérèse.

Les Demanderesses ont mis au point une solution technique qui permet d'atteindre un compromis amélioré entre la cohésion, la rigidité et l'hystérèse de la composition de caoutchouc.

L'invention a pour objet une composition de caoutchouc à base d'au moins un élastomère diénique, une charge inorganique renforçante et un composé 1,3-dipolaire choisi dans le groupe constitué par les oxydes de nitrile, les imines de nitrile et les nitrones, caractérisée en ce que le composé 1,3-dipolaire répond à la formule (I)

Q-A-B (1)

Q contenant un motif -C≡N→O, -C≡N→N- ou -C=N(->O)
A, de préférence divalent, est un atome ou un groupe d'atomes reliant Q à B,
B représente une fonction ester d'acide carboxylique répondant à la formule (II)

   -C(OR)=O (II)

   dans laquelle R est un groupe carboné pouvant contenir au moins un hétéroatome.

L'invention a aussi pour objet une bande de roulement comprenant la composition de caoutchouc conforme à l'invention.

L'invention a encore pour objet un pneumatique comprenant la composition de caoutchouc conforme à l'invention, notamment dans sa bande de roulement.

### I. DESCRIPTION DETAILLEE DE L'INVENTION

Dans la présente description, sauf indication expresse différente, tous les pourcentages (%) indiqués sont des % en masse. L'abréviation "pce" signifie parties en poids pour cent parties d'élastomère (du total des élastomères si plusieurs élastomères sont présents).

D'autre part, tout intervalle de valeurs désigné par l'expression "entre a et b" représente le domaine de valeurs supérieur à "a" et inférieur à "b" (c'est-à-dire bornes a et b exclues) tandis que tout intervalle de valeurs désigné par l'expression "de a à b" signifie le domaine de valeurs allant de "a" jusqu'à "b" (c'est-à-dire incluant les bornes strictes a et b).

Par l'expression composition "à base de", il faut entendre dans la présente description une composition comportant le mélange et/ou le produit de réaction in situ des différents constituants utilisés, certains de ces constituants de base (par exemple l'élastomère, la charge ou autre additif classiquement utilisé dans une composition de caoutchouc destinée à la fabrication de pneumatique) étant susceptibles de, ou destinés à réagir entre eux, au moins en partie, lors des différentes phases de fabrication de la composition destinée à la fabrication de pneumatique.

Le composé 1,3-dipolaire répondant à la formule (I) a pour caractéristique essentielle de comprendre un groupe Q et un groupe B reliés entre eux par un groupe A dans lequel :
Q contient un motif -C≡N→O, -C≡N→N- ou -C=N(->O),
B représente une fonction ester d'acide carboxylique,
A, de préférence divalent, est un atome ou un groupe d'atomes reliant Q à B.

   Q-A-B (I)

Le terme composé 1,3-dipolaire est compris selon la définition donnée par IUPAC.

Selon l'invention, le groupe B répond à la formule (II)

-C(OR)=O (II)

dans laquelle R est un groupe carboné pouvant contenir au moins un hétéroatome.

Le groupe carboné pouvant contenir un hétéroatome contient de préférence 1 à 20 atomes de carbone, de manière plus préférentielle 1 à 12 atomes de carbone, de manière encore plus préférentielle 1 à 6 atomes de carbone. Selon l'un quelconque mode de réalisation de l'invention, conviennent comme R groupe carboné ceux ayant de 1 à 3 atomes de carbone tels que le groupe méthyle ou le groupe éthyle.

A peut être un groupe contenant jusqu'à 20 atomes de carbone, lequel groupe peut contenir au moins un hétéroatome. A peut être un groupe aliphatique ou aromatique.

Lorsque A est un groupe aliphatique, A contient préférentiellement 1 à 20 atomes de carbone, plus préférentiellement 1 à 12 atomes de carbone, encore plus préférentiellement 1 à 6 atomes de carbone, tout particulièrement 1 à 3 atomes de carbone. Lorsque A est un groupe aromatique, A contient préférentiellement 6 à 20 atomes de carbone, plus préférentiellement 6 à 12 atomes de carbone.

Comme groupe divalent A convient particulièrement un groupe alkylène contenant 1 à 20 atomes de carbone, préférentiellement 1 à 12 atomes de carbone, plus préférentiellement 1 à 6 atomes de carbone, encore plus préférentiellement 1 à 3 atomes de carbone. On peut citer comme groupe A divalent contenant 1 à 3 atomes de carbone qui convient le groupe méthylène.

Comme groupe divalent A peut aussi convenir un groupe arylène contenant de préférence 6 à 20 atomes de carbone, plus préférentiellement 6 à 12 atomes de carbone.

Conviennent comme composés 1,3-dipolaires les composés choisis dans le groupe constitué par les oxydes de nitrile, les imines de nitrile et les nitrones, auquel cas Q contient un motif -C≡N→O, -C≡N→N- ou -C=N(→O)-.

Selon le mode de réalisation particulier de l'invention où Q comprend un motif -C≡N→O, Q comporte préférentiellement le motif répondant à la formule (III) dans laquelle quatre des cinq symboles R1 à R5 identiques ou différents, sont chacun un atome ou un groupe d'atomes et le cinquième symbole désigne un rattachement direct ou indirect à A, sachant que R1 et R5 sont tous les deux différents de H. Les quatre des cinq symboles R1 à R5 peuvent être des groupes aliphatiques ou aromatiques. Les groupes aliphatiques peuvent contenir 1 à 20 atomes de carbone, préférentiellement 1 à 12 atomes de carbone, plus préférentiellement 1 à 6 atomes de carbone, encore plus préférentiellement 1 à 3 atomes de carbone. Les groupes aromatiques peuvent contenir 6 à 20 atomes de carbone, préférentiellement 6 à 12 atomes de carbone.

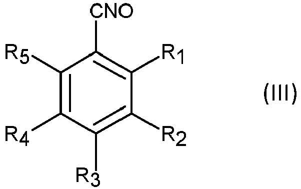

R1, R3 et R5 sont préférentiellement chacun un groupe alkyle de 1 à 6 atomes de carbone, plus préférentiellement de 1 à 3 atomes de carbone, encore plus préférentiellement un groupe méthyle ou éthyle.

Selon une variante de ce mode de réalisation particulier de l'invention, R1, R3 et R5 sont identiques. Selon cette variante où ils sont identiques, R1, R3 et R5 sont préférentiellement chacun un groupe alkyle de 1 à 6 atomes de carbone, plus préférentiellement de 1 à 3 atomes de carbone, encore plus préférentiellement un groupe méthyle ou éthyle.

Dans le cas où le cinquième symbole désigne un rattachement indirect à A, le cinquième symbole représente de préférence un hétéroatome, de préférence l'oxygène.

De manière davantage préférentielle, le composé 1,3-dipolaire est le composé 3-(2-éthoxy-2-oxoéthoxy)-2,4,6-triméthylbenzonitrile oxide répondant à la formule (IIIa)

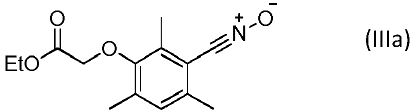

Selon le mode de réalisation particulier de l'invention où Q comprend un motif -C=N(→O)-, Q comporte préférentiellement le motif répondant à la formule (IV) ou (V) 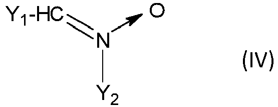 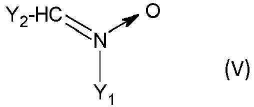 dans laquelle :
Y₁ est un groupe aliphatique, préférentiellement un groupe alkyle contenant de préférence 1 à 12 atomes de carbone, ou un groupe aromatique contenant 6 à 20 atomes de carbone, préférentiellement un groupe alkylaryle, plus préférentiellement un groupe phényle ou tolyle,
et Y₂ est un groupe aliphatique, préférentiellement un groupe hydrocarboné saturé contenant de préférence 1 à 12 atomes de carbone et comportant un rattachement direct à A, ou un groupe aromatique contenant préférentiellement 6 à 20 atomes de carbone et comportant sur son noyau benzénique un rattachement direct à A.

Une caractéristique essentielle de la composition de caoutchouc conforme à l'invention est de comprendre le composé 1,3-dipolaire défini selon l'un quelconque mode de réalisation de l'invention.

La quantité de composé 1,3-dipolaire introduite dans la composition de caoutchouc est exprimée en équivalent molaire de fonction ester d'acide carboxylique. Par exemple, si le composé 1,3-dipolaire contient une seule fonction ester d'acide carboxylique, à une mole de composé 1,3-dipolaire correspond une mole de fonction ester d'acide carboxylique. Si le composé 1,3-dipolaire contient deux fonctions ester d'acide carboxylique, à une mole de composé 1,3-dipolaire correspond deux moles de fonctions d'ester d'acide carboxylique. Dans ce dernier cas l'utilisation du composé 1,3-dipolaire selon un équivalent molaire de fonction ester d'acide carboxylique correspond à une demi-mole de composé 1,3-dipolaire.

Selon l'un quelconque mode de réalisation de l'invention, la quantité de composé 1,3-dipolaire dans la composition de caoutchouc est préférentiellement comprise entre 0 et 5 équivalents molaires, plus préférentiellement entre 0 et 2 équivalents molaires, encore plus préférentiellement entre 0 et 1 équivalent molaire de fonction ester d'acide carboxylique pour 100 moles d'unités monomères constituant l'élastomère diénique. Ces plages préférentielles permettent d'optimiser de façon plus fine le compromis entre la cohésion et l'hystérèse de la composition de caoutchouc, notamment dans un pneumatique. Pour chacune de ces plages préférentielles, la borne inférieure est de préférence d'au moins 0.1 équivalent molaire de composé 1,3-dipolaire.

Par élastomère (ou indistinctement caoutchouc) "diénique", doit être compris de manière connue un (ou plusieurs) élastomère constitué au moins en partie (i.e., un homopolymère ou un copolymère) d'unités monomères diènes (monomères porteurs de deux doubles liaisons carbone-carbone, conjuguées ou non).

Ces élastomères diéniques peuvent être classés dans deux catégories : "essentiellement insaturés" ou "essentiellement saturés". On entend en général par "essentiellement insaturé", un élastomère diénique issu au moins en partie de monomères diènes conjugués, ayant un taux de motifs ou unités d'origine diénique (diènes conjugués) qui est supérieur à 15% (% en moles) ; c'est ainsi que des élastomères diéniques tels que les caoutchoucs butyle ou les copolymères de diènes et d'alpha-oléfines type EPDM n'entrent pas dans la définition précédente et peuvent être notamment qualifiés d'élastomères diéniques "essentiellement saturés" (taux de motifs d'origine diénique faible ou très faible, toujours inférieur à 15%). Dans la catégorie des élastomères diéniques "essentiellement insaturés", on entend en particulier par élastomère diénique "fortement insaturé" un élastomère diénique ayant un taux de motifs d'origine diénique (diènes conjugués) qui est supérieur à 50%.

Ces définitions étant données, on entend plus particulièrement par élastomère diénique susceptible d'être utilisé dans les compositions conformes à l'invention:
(a) - tout homopolymère d'un monomère diène conjugué, notamment tout homopolymère obtenu par polymérisation d'un monomère diène conjugué ayant de 4 à 12 atomes de carbone;
(b) - tout copolymère obtenu par copolymérisation d'un ou plusieurs diènes conjugués entre eux ou avec un ou plusieurs composés vinyle aromatique ayant de 8 à 20 atomes de carbone;
(c) - un copolymère ternaire obtenu par copolymérisation d'éthylène, d'une α-oléfine ayant de 3 à 6 atomes de carbone avec un monomère diène non conjugué ayant de 6 à 12 atomes de carbone, comme par exemple les élastomères obtenus à partir d'éthylène, de propylène avec un monomère diène non conjugué du type précité tel que notamment l'hexadiène-1,4, l'éthylidène norbornène, le dicyclopentadiène;
(d) - un copolymère d'isobutène et d'isoprène (caoutchouc butyle), ainsi que les versions halogénées, en particulier chlorées ou bromées, de ce type de copolymère.

Bien qu'elle s'applique à tout type d'élastomère diénique, l'homme du métier du pneumatique comprendra que la présente invention est de préférence mise en oeuvre avec des élastomères diéniques essentiellement insaturés, en particulier du type (a) ou (b) ci-dessus.

Dans le cas de copolymères du type (b), ceux-ci contiennent de 20 à 99% en poids d'unités diéniques et de 1 à 80% en poids d'unités vinylaromatique.

A titre de diènes conjugués conviennent notamment le butadiène-1,3, le 2-méthyl-1,3-butadiène, les 2,3-di(alkyle en C₁-C₅)-1,3-butadiènes tels que par exemple le 2,3-diméthyl-1,3-butadiène, le 2,3-diéthyl-1,3-butadiène, le 2-méthyl-3-éthyl-1,3-butadiène, le 2-méthyl-3-isopropyl-1,3-butadiène, un aryl-1,3-butadiène, le 1,3-pentadiène, le 2,4-hexadiène.

A titre de composés vinylaromatiques conviennent par exemple le styrène, l'ortho-, méta-, para-méthylstyrène, le mélange commercial "vinyle-toluène", le para-tertiobutylstyrène, les méthoxystyrènes, les chlorostyrènes, le vinylmésitylène, le divinylbenzène, le vinylnaphtalène.

Préférentiellement, l'élastomère diénique est un élastomère essentiellement insaturé choisi dans le groupe constitué par les polybutadiènes, les polyisoprènes, les copolymères de butadiène, les copolymères d'isoprène, et les mélanges de ces élastomères. A titre d'élastomère diénique convient tout particulièrement un polybutadiène (BR), un copolymère de butadiène et de styrène (SBR), un caoutchouc naturel (NR) ou un polyisoprène de synthèse (IR) présentant préférentiellement un taux molaire de liaison cis-1,4 supérieur à 90%.

La composition de caoutchouc conforme à l'invention comporte une charge inorganique renforçante.

Par "charge inorganique renforçante", doit être entendu ici toute charge inorganique ou minérale, quelles que soient sa couleur et son origine (naturelle ou de synthèse), encore appelée charge "blanche", charge "claire" ou même charge "non-noire" par opposition au noir de carbone, capable de renforcer à elle seule, sans autre moyen qu'un agent de couplage intermédiaire, une composition de caoutchouc destinée à la fabrication de bandages pneumatiques, en d'autres termes apte à remplacer, dans sa fonction de renforcement, un noir de carbone conventionnel de grade pneumatique ; une telle charge se caractérise généralement, de manière connue, par la présence de groupes hydroxyle (-OH) à sa surface.

Comme charges inorganiques renforçantes conviennent notamment des charges minérales du type siliceuse, préférentiellement la silice (SiOz). La silice utilisée peut être toute silice renforçante connue de l'homme du métier, notamment toute silice précipitée ou pyrogénée présentant une surface BET ainsi qu'une surface spécifique CTAB toutes deux inférieures à 450 m²/g, de préférence de 30 à 400 m²/g, notamment entre 60 et 300 m²/g. A titres de silices précipitées hautement dispersibles (dites "HDS"), on citera par exemple les silices « Ultrasil » 7000 et « Ultrasil » 7005 de la société Degussa, les silices « Zeosil » 1165MP, 1135MP et 1115MP de la société Rhodia, la silice « Hi-Sil » EZ150G de la société PPG, les silices « Zeopol » 8715, 8745 et 8755 de la Société Huber, les silices à haute surface spécifique telles que décrites dans la demande WO 03/016387.

Dans le présent exposé, la surface spécifique BET est déterminée de manière connue par adsorption de gaz à l'aide de la méthode de Brunauer-Emmett-Teller décrite dans *"*The Journal of the American Chemical Society" Vol. 60, page 309, février 1938, plus précisément selon la norme française NF ISO 9277 de décembre 1996 (méthode volumétrique multipoints (5 points) - gaz: azote - dégazage: 1heure à 160°C - domaine de pression relative *p*/*po* : 0.05 à 0.17). La surface spécifique CTAB est la surface externe déterminée selon la norme française NF T 45-007 de novembre 1987 (méthode B).

L'état physique sous lequel se présente la charge inorganique renforçante est indifférent, que ce soit sous forme de poudre, de microperles, de granulés, ou encore de billes. Bien entendu on entend également par charge inorganique renforçante des mélanges de différentes charges inorganiques renforçantes, en particulier de silices hautement dispersibles telles que décrites ci-dessus.

L'homme du métier comprendra qu'à titre de charge équivalente de la charge inorganique renforçante décrite dans le présent paragraphe, pourrait être utilisée une charge renforçante d'une autre nature, notamment organique telle que du noir de carbone, dès lors que cette charge renforçante serait recouverte d'une couche inorganique telle que silice, ou bien comporterait à sa surface des sites fonctionnels, notamment hydroxyles, nécessitant l'utilisation d'un agent de couplage pour établir la liaison entre la charge et l'élastomère. A titre d'exemple, on peut citer par exemple des noirs de carbone pour pneumatiques tels que décrits par exemple dans les documents brevet WO 96/37547, WO 99/28380.

Selon un mode de réalisation particulier de l'invention, la charge inorganique, préférentiellement une silice, représente plus de 50% en masse de la masse de la charge renforçante de la composition de caoutchouc. On dit alors que la charge inorganique renforçante est majoritaire.

Lorsqu'il est combiné à une charge inorganique renforçante majoritaire telle que la silice, le noir de carbone est utilisé de préférence à un taux inférieur à 20 pce, plus préférentiellement inférieur à 10 pce (par exemple entre 0.5 et 20 pce, notamment entre 2 et 10 pce). Dans les intervalles indiqués, on bénéficie des propriétés colorantes (agent de pigmentation noire) et anti-UV des noirs de carbone, sans pénaliser par ailleurs les performances typiques apportées par la charge inorganique renforçante.

Comme noirs de carbone conviennent tous les noirs de carbone, notamment les noirs conventionnellement utilisés dans les pneumatiques ou leurs bandes de roulement (noirs dits de grade pneumatique). Parmi ces derniers, on citera plus particulièrement les noirs de carbone renforçants des séries 100, 200, 300, ou les noirs de série 500, 600 ou 700 (grades ASTM), comme par exemple les noirs N115, N134, N234, N326, N330, N339, N347, N375, N550, N683, N772). Ces noirs de carbone peuvent être utilisés à l'état isolé, tels que disponibles commercialement, ou sous tout autre forme, par exemple comme support de certains des additifs de caoutchouterie utilisés.

Le taux de charge inorganique renforçante est compris préférentiellement entre 30 et 160 pce, plus préférentiellement entre 40 pce et 160 pce. En deçà de 30 pce, le renforcement de la composition de caoutchouc peut être insuffisant pour apporter un niveau de cohésion ou de résistance à l'usure adéquats du composant caoutchouteux du pneumatique comprenant cette composition. De manière encore plus préférentielle, le taux de charge inorganique renforçante est d'au moins 50 pce. Au-delà de 160 pce, il existe un risque d'augmentation de l'hystérèse et donc de la résistance au roulement des pneumatiques. Pour cette raison, le taux de charge inorganique renforçante est de préférence dans un domaine allant de 50 à 120 pce, notamment pour un usage dans une bande de roulement de pneumatique. L'une quelconque de ces plages de taux de charge inorganique renforçante s'applique à l'un quelconque des modes de réalisation de l'invention.

Pour coupler la charge inorganique renforçante à l'élastomère diénique, on utilise de manière bien connue un agent de couplage, notamment un silane, (ou agent de liaison) au moins bifonctionnel destiné à assurer une connexion suffisante, de nature chimique et/ou physique, entre la charge inorganique (surface de ses particules) et l'élastomère diénique. On utilise en particulier des organosilanes ou des polyorganosiloxanes au moins bifonctionnels.

On utilise notamment des silanes polysulfurés, dits "symétriques" ou "asymétriques" selon leur structure particulière, tels que décrits par exemple dans les demandes WO03/002648 (ou US 2005/016651) et WO03/002649 (ou US 2005/016650).

Conviennent en particulier, sans que la définition ci-après soit limitative, des silanes polysulfurés répondant à la formule générale (V)

Z - A - Sₓ - A - Z (V)

dans laquelle :
- x est un entier de 2 à 8 (de préférence de 2 à 5) ;
- les symboles A, identiques ou différents, représentent un radical hydrocarboné divalent (de préférence un groupement alkylène en C₁-C₁₈ ou un groupement arylène en C₆-C₁₂, plus particulièrement un alkylène en C₁-C₁₀, notamment en C₁-C₄, en particulier le propylène) ;
- les symboles Z, identiques ou différents, répondent à l'une des trois formules ci-après: 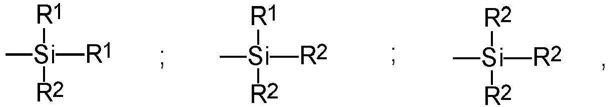 dans lesquelles:
   - les radicaux R¹, substitués ou non substitués, identiques ou différents entre eux, représentent un groupe alkyle en C₁-C₁₈, cycloalkyle en C₅-C₁₈ ou aryle en C₆-C₁₈ (de préférence des groupes alkyle en C₁-C₆, cyclohexyle ou phényle, notamment des groupes alkyle en C₁-C₄, plus particulièrement le méthyle et/ou l'éthyle).
   - les radicaux R², substitués ou non substitués, identiques ou différents entre eux, représentent un groupe alkoxyle en C₁-C₁₈ ou cycloalkoxyle en C₅-C₁₈ (de préférence un groupe choisi parmi alkoxyles en C₁-C₈ et cycloalkoxyles en C₅-C₈, plus préférentiellement encore un groupe choisi parmi alkoxyles en C₁-C₄, en particulier méthoxyle et éthoxyle).

Dans le cas d'un mélange d'alkoxysilanes polysulfurés répondant à la formule (I) ci-dessus, notamment des mélanges usuels disponibles commercialement, la valeur moyenne des "x" est un nombre fractionnaire de préférence compris entre 2 et 5, plus préférentiellement proche de 4. Mais l'invention peut être aussi avantageusement mise en oeuvre par exemple avec des alkoxysilanes disulfurés (x = 2).

A titre d'exemples de silanes polysulfurés, on citera plus particulièrement les polysulfures (notamment disulfures, trisulfures ou tétrasulfures) de bis-(alkoxyl(C₁-C₄)-alkyl(C₁-C₄)silyl-alkyl(C₁-C₄)), comme par exemple les polysulfures de bis(3-triméthoxysilylpropyl) ou de bis(3-triéthoxysilylpropyl). Parmi ces composés, on utilise en particulier le tétrasulfure de bis(3-triéthoxysilylpropyl), en abrégé TESPT, de formule [(C₂H₅O)₃Si(CH₂)₃S₂]₂ ou le disulfure de bis-(triéthoxysilylpropyle), en abrégé TESPD, de formule [(C₂H₅O)₃Si(CH₂)₃S]₂.

A titre d'agent de couplage autre qu'alkoxysilane polysulfuré, on citera notamment des POSS (polyorganosiloxanes) bifonctionnels ou encore des polysulfures d'hydroxysilane tels que décrits dans les demandes de brevets WO 02/30939 (ou US 6,774,255), WO 02/31041 (ou US 2004/051210) ou encore des silanes ou POSS porteurs de groupements fonctionnels azo-dicarbonyle, tels que décrits par exemple dans les demandes de brevets WO 2006/125532, WO 2006/125533, WO 2006/125534.

La teneur en agent de couplage est avantageusement inférieure à 20 pce, étant entendu qu'il est en général souhaitable d'en utiliser le moins possible. Typiquement le taux d'agent de couplage représente de 0,5% à 15% en poids par rapport à la quantité de charge inorganique. Son taux est préférentiellement compris entre 0,5 et 12 pce, plus préférentiellement compris dans un domaine allant de 3 à 10 pce. Ce taux est aisément ajusté par l'homme du métier selon le taux de charge inorganique utilisé dans la composition.

La composition de caoutchouc conforme à l'invention peut également contenir, en complément des agents de couplage, des activateurs de couplage, des agents de recouvrement des charges inorganiques ou plus généralement des agents d'aide à la mise en oeuvre susceptibles de manière connue, grâce à une amélioration de la dispersion de la charge dans la matrice de caoutchouc et à un abaissement de la viscosité des compositions, d'améliorer leur faculté de mise en oeuvre à l'état cru.

La composition de caoutchouc conforme à l'invention peut comporter également tout ou partie des additifs usuels habituellement utilisés dans les compositions d'élastomères destinées à constituer des mélanges externes d'articles finis en caoutchouc tels que des pneumatiques, en particulier de bandes de roulement, comme par exemple des plastifiants ou des huiles d'extension, que ces derniers soient de nature aromatique ou non-aromatique, notamment des huiles très faiblement ou non aromatiques (e.g., huiles paraffiniques, naphténiques hydrogénées, huiles MES ou TDAE), des huiles végétales, en particulier les esters de glycérol comme les trioléates de glycérol, des résines plastifiantes hydrocarbonées présentant une haute Tg, de préférence supérieure à 30°C, telles que décrites par exemple dans les demandes WO 2005/087859, WO 2006/061064 et WO 2007/017060, des pigments, des agents de protection tels que cires anti-ozone, anti-ozonants chimiques, anti-oxydants, des agents anti-fatigue, des résines renforçantes (tels que résorcinol ou bismaléimide), des accepteurs (par exemple résine phénolique novolaque) ou des donneurs de méthylène (par exemple HMT ou H3M) tels que décrits par exemple dans la demande WO 02/10269, un système de réticulation, des accélérateurs ou retardateurs de vulcanisation, des activateurs de vulcanisation. Le système de réticulation est de préférence à base de soufre, mais il peut être également à base de donneurs de soufre, de peroxyde, de bismaléimides ou de leurs mélanges.

La composition de caoutchouc conforme à l'invention est fabriquée dans des mélangeurs appropriés, en utilisant deux phases de préparation successives bien connues de l'homme du métier : une première phase de travail ou malaxage thermomécanique (phase dite « non-productive ») à haute température, jusqu'à une température maximale comprise entre 130°C et 200°C, suivie d'une seconde phase de travail mécanique (phase dite « productive ») jusqu'à une plus basse température, typiquement inférieure à 110°C, par exemple entre 40°C et 100°C, phase de finition au cours de laquelle est incorporé le système de réticulation.

Le procédé pour préparer la composition de caoutchouc conforme à l'invention et à base d'au moins un système de réticulation comprend les étapes suivantes :
- ajouter au cours d'une première étape dite non productive à l'élastomère diénique le composé 1,3-dipolaire, la charge inorganique renforçante, le cas échéant l'agent de couplage, en malaxant thermomécaniquement jusqu'à atteindre une température maximale comprise entre 130 et 200°C,
- refroidir l'ensemble à une température inférieure à 100°C,
- incorporer ensuite le système de réticulation,
- malaxer le tout jusqu'à une température maximale inférieure à 120°C.

Selon un mode de réalisation préférentiel de l'invention, le composé 1,3-dipolaire est incorporé à l'élastomère diénique avant l'introduction des autres constituants de la composition de caoutchouc. Le temps de contact entre l'élastomère diénique et le composé 1,3-dipolaire qui sont malaxés thermomécaniquement est ajusté en fonction des conditions du malaxage thermomécanique, notamment en fonction de la température. Plus la température du malaxage est élevée, plus ce temps de contact est court. Typiquement il est de 1 à 5 minutes pour une température de 100 à 130°C.

Selon ce mode de réalisation préférentiel de l'invention, on ajoute de préférence au moins un antioxydant à l'élastomère diénique avant son introduction dans un mélangeur, notamment à la fin de la synthèse de l'élastomère diénique comme cela se fait conventionnellement.

Après l'incorporation de tous les ingrédients de la composition de caoutchouc, la composition finale ainsi obtenue est ensuite calandrée, par exemple sous la forme d'une feuille ou d'une plaque, notamment pour une caractérisation au laboratoire, ou encore extrudée, pour former par exemple un profilé de caoutchouc utilisé comme composant caoutchouteux pour la confection du pneumatique.

Ainsi selon un mode de réalisation particulier de l'invention, la composition de caoutchouc conforme à l'invention, pouvant être soit à l'état cru (avant réticulation ou vulcanisation), soit à l'état cuit (après réticulation ou vulcanisation), est dans un pneumatique, notamment dans une bande de roulement de pneumatique.

Les caractéristiques précitées de la présente invention, ainsi que d'autres, seront mieux comprises à la lecture de la description suivante de plusieurs exemples de réalisation de l'invention, donnés à titre illustratif et non limitatif.

### II. EXEMPLES DE REALISATION DE L'INVENTION

### 11.1-Mesures et tests utilisés :

### Analyse RMN :

L'analyse structurale ainsi que la détermination des puretés molaires des molécules de synthèses sont réalisées par une analyse RMN. Les spectres sont acquis sur un spectromètre Avance 3 400 MHz BRUKER équipé d'une sonde " large bande " BBFO-zgrad 5 mm. L'expérience RMN ¹H quantitative, utilise une séquence simple impulsion 30° et un délai de répétition de 3 secondes entre chacune des 64 acquisitions. Les échantillons sont solubilisés dans le Diméthylsulfoxide deutéré (DMSO). Ce solvant est également utilisé pour le signal de lock. La calibration est réalisée sur le signal des protons du DMSO deutéré à 2.44 ppm et sur les carbones du DMSO deutéré à 39.5 ppm par rapport à une référence TMS à 0ppm. Le spectre RMN ¹H couplé aux expériences 2D HSQC ¹H/13C et HMBC ¹H/¹³C permettent la détermination structurale des molécules (cf tableaux d'attributions). Les quantifications molaires sont réalisées à partir du spectre RMN 1D ¹H quantitatif.

### Essais de traction :

Ces essais de traction permettent de déterminer les contraintes d'élasticité. Sauf indication différente, ils sont effectués conformément à la norme française NF T 46-002 de septembre 1988. Un traitement des enregistrements de traction permet également de tracer la courbe de module en fonction de l'allongement. On mesure en première élongation les modules sécants nominaux calculés en se ramenant à la section initiale de l'éprouvette (ou contrainte apparente, en MPa) à 100% et 300% d'allongement notés MSA100 et MSA300 respectivement.

Toutes ces mesures de traction sont effectuées dans les conditions normales de température (23 ± 2°C) selon la norme NF T 46-002.

Les modules MSA100 et MSA300 sont exprimés en base 100 par rapport à la composition témoin. Une valeur supérieure à 100 traduit une augmentation du module.

L'indice de renforcement qui est le rapport du module MSA300 sur le module MSA100, est exprimé en base 100 par rapport à la composition témoin. Une valeur supérieure à 100 traduit une amélioration du renforcement de la composition considérée par rapport à la composition témoin.

### Propriétés dynamiques :

Les propriétés dynamiques tan(δ)max sont mesurées sur un viscoanalyseur (Metravib VA4000), selon la norme ASTM D 5992-96. On enregistre la réponse d'un échantillon de composition vulcanisée (éprouvette cylindrique de 4 mm d'épaisseur et de 400 mm² de section), soumis à une sollicitation sinusoïdale en cisaillement simple alterné, à la fréquence de 10Hz, dans les conditions normales de température (23°C) selon la norme ASTM D 1349-99. On effectue un balayage en amplitude de déformation de 0,1% à 100% (cycle aller), puis de 100% à 0,1% (cycle retour). Les résultats exploités sont l'écart de module (ΔG*) entre les valeurs à 0,1 et 100% de déformation (effet Payne), le module complexe de cisaillement dynamique (G*) à 25% de déformation et la valeur maximale de tanδ observée pour le cycle retour, notée tan(δ)max.

Les valeurs de ΔG^{∗} sont exprimées en base 100 par rapport au témoin : une valeur inférieure à 100 traduit une diminution de l'écart de module, soit une augmentation de la linéarisation de la composition de caoutchouc.

Les valeurs de G* sont exprimées en base 100 par rapport au témoin : une valeur supérieure à 100 traduit une augmentation du module, soit une augmentation de la rigidité de la composition de caoutchouc

Les valeurs de tan(δ)max sont exprimées en base 100 par rapport au témoin : une valeur inférieure à 100 traduit une diminution de la valeur maximale du facteur de perte, soit une diminution de l'hystérèse de la composition de caoutchouc.

### 11.2-Synthèse du composé 1,3-dipolaire 3-(2-ethoxy-2-oxoethoxy)-2,4,6-trimethylbenzonitrile oxide :

Ce composé peut être préparé selon le schéma réactionnel suivant :

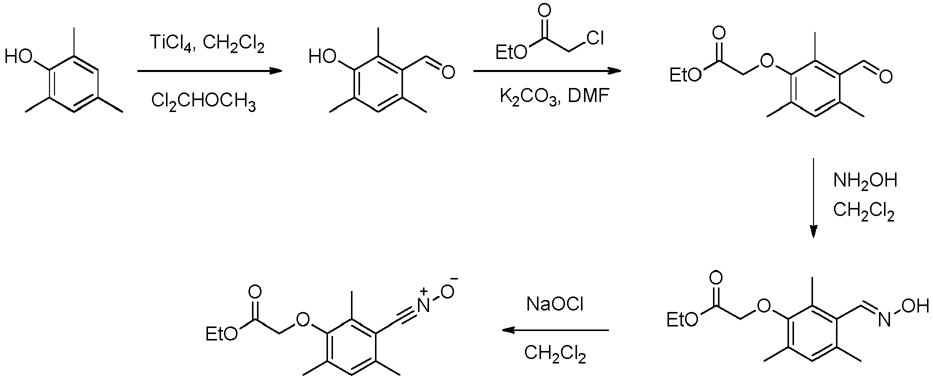

La préparation du composé 3-hydroxy-2,4,6-trimethylbenzaldéhyde est décrite dans l'article Yabukov, A. P. ; Tsyganov, D.V. ; Belen'kii, L.I. ; Krayushkin, M.M. ; Bulletin of the Academy of Sciences of the USSR, Division od Chemical Science (English Translation) ; vol. 40; nb 7.2; (1991) ; p. 1427-1432*;* Izvestiya Akademii Nauk SSSR, Seriya Khimicheskaya ; nb. 7 (1991); p 1609-1615*.*

### II.2-1-Synthèse de l'éthyl-2-(3-formyl-2,4,6-triméthylphénoxy)acétate:

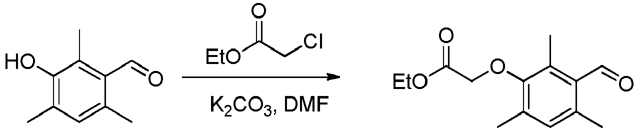

Un mélange de 3-hydroxy-2,4,6-triméthylbenzaldéhyde (30,00 g, 0,183 mol.) et K₂CO₃ (18,94 g, 0,137 mol) dans le DMF (110 ml) est agité à température ambiante pendant 10-15 minutes. A ce mélange est ajouté l'éthyle de chloroacétate (22,4 g, 0,183 mol.) dans le DMF (15 ml). La température du mélange est portée à 74°C pendant 4 heures. Après retour à température ambiante, le mélange est dilué par l'eau (800 ml) et CH₂Cl₂ (150 ml). La phase aqueuse est extraite par CH₂Cl₂ (3 fois par 50 ml). Les phases organiques sont rassemblées puis lavées par une solution de NaOH (5,0 g, 0,125 mol) dans l'eau (100 ml), lavées par l'eau (4 fois par 100 ml) et concentrées sous pression réduite jusqu'à 21 mbar (36°C). Un solide blanc (42,96 g, rendement 94 %) de point de fusion 55 °C est obtenu. La pureté molaire est supérieure à 98 % (RMN ¹H).

| 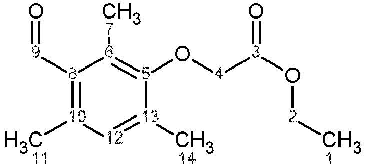 | | |
|---|---|---|
| N° | **δ ₁H (ppm)** | **δ ₁₃C (ppm)** |
| 1 | 1.18 | 14.4 |
| 2 | 4.13 | 60.9 |
| 3 | / | 168.8 |
| 4 | 4.37 | 49.5 |
| 5 | / | 153.9 |
| 6/10/13 | / | Entre 130 et 140 |
| 7 | 2.39 | 12.4 |
| 8 | / | 131.7 |
| 9 | 10.36 | 194.1 |
| 11 | 2.39 | 19.7 |
| 12 | 6.94 | 132.1 |
| 14 | 2.20 | 16.7 |

### II.2-2-Synthèse de l'éthyl-2-(3-((hydroxyimino)méthyl)-2,4,6-triméthylphenoxy)acétate :

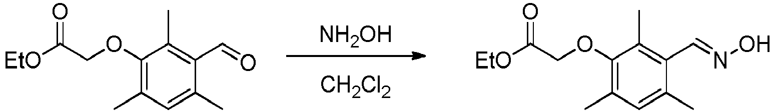

A une solution d'éthyl-2-(3-formyl-2,4,6-triméthylphénoxy)acétate (42,5 g, 0,170 mol) dans EtOH (250 ml) à 40°C est ajoutée une solution aqueuse d'hydroxylamine (13,5 g, 0,204 mol, 50 % dans l'eau, Aldrich) dans EtOH (25 ml). Le milieu réactionnel est ensuite agité à une température comprise entre 45°C et 50°C. Après 4 heures à cette température, de l'eau (50 ml) est ajoutée au milieu réactionnel. Le milieu réactionnel est évaporé sous pression réduite (T_{bain} 37 ºC, 80 mbar) jusqu'à obtention d'une suspension. Le précipité obtenu est filtré et lavé sur le filtre par EtOH/eau (15 ml / 45 ml), puis par EtOH / éther de pétrole (15 ml / 45 ml) et enfin par l'éther de pétrole (2×30 ml). Le produit obtenu est séché sous pression atmosphérique à température ambiante. Un solide blanc (31,83 g, rendement 73 %) de point de fusion 89 °C est obtenu. La pureté molaire est supérieure à 99 % (RMN ¹H). 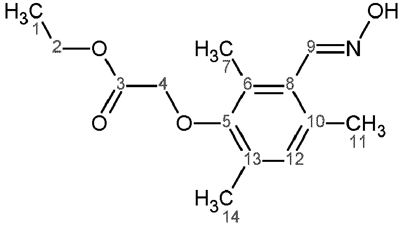 Solvant : DMSO

| N° | **δ ₁H (ppm)** | **δ ₁₃C (ppm)** |
|---|---|---|
| 1 | 1.17 | 13.8 |
| 2 | 4.13 | 60.2 |
| 3 | / | 168.5 |
| 4 | 4.34 | 68.8 |
| 5 | / | 152.9 |
| 6/8/10/13 | / | Entre 129.2 et 132.2 |
| 7 | 2.17 | 13.2 |
| 9 | 8.19 | 147.3 |
| 11 | 2.18 | 20.1 |
| 12 | 6.86 | 130.3 |
| 14 | 2.14 | 15.6 |

### II.2-3-Synthèse du 3-(2-éthoxy-2-oxoéthoxy)-2,4,6-triméthylbenzonitrile oxide :

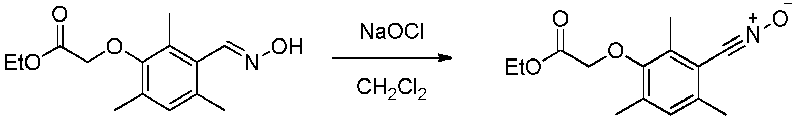

Une solution d'éthyl-2-(3-((hydroxyimino)méthyl)-2,4,6-triméthylphenoxy)acétate (20,0 g, 0,075 mol) dans CH₂Cl₂ (450 ml) est refroidie jusqu'à - 4°C. Une fois à cette température, de l'eau de Javel (4 % du chlore actif, Aldrich) (92 ml) est ajoutée goutte à goutte pendant 5 minutes. La température du milieu réactionnel pendant l'addition est maintenue entre -4°C et - 1°C. Le milieu réactionnel est ensuite agité pendant 35 minutes entre 0 et 5°C puis agité jusqu'à retour à température ambiante (3,0 - 3,5 heures). La phase aqueuse est séparée et lavée par CH₂Cl₂ (3 fois par 20 ml). Les phases organiques rassemblées sont lavées par l'eau (3 fois par 75 mL) et concentrées sous pression réduite (Tbain 25 ºC) jusqu'à 50 ml (97 g). La solution obtenue est diluée par l'éther de pétrole (120 ml, fractions 40/60°C) et concentrée sous pression réduite (Tbain 25 ºC) jusqu'à formation d'un précipité. Après 4-5 heures à - 18°C, le précipité est filtré et lavé sur le filtre par un mélange CH₂Cl₂/ l'éther de pétrole (5 ml / 20 ml), puis par l'éther de pétrole (2 fois par 25 ml, fractions 40/60°C) et enfin séché pendant 12 heures sous pression atmosphérique à température ambiante. Un solide blanc (13,67 g, 0,052 mol, rendement 69 %) de point de fusion 102 °C est obtenu. La pureté molaire est supérieure à 99 % (RMN ¹H).

| 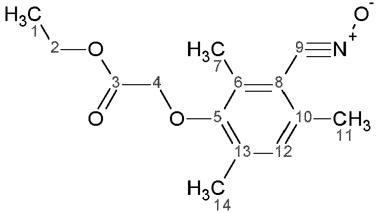 | | |
|---|---|---|
| N° | **δ ₁H (ppm)** | **δ ₁₃C (ppm)** |
| 1 | 1.17 | 13.7 |
| 2 | 4.13 | 60.3 |
| 3 | / | 168.3 |
| 4 | 4.41 | 68.8 |
| 5 | / | 152.8 |
| 6/10 | / | 133.8/137.1 |
| 7 | 2.27 | 14.3 |
| 8 | / | 111.8 |
| 9 | / | / |
| 11 | 2.27 | 19.5 |
| 12 | 7.01 | 130.0 |
| 13 | / | 134.3 |
| 14 | 2.18 | 16.0 |

### II.3-Préparation des compositions de caoutchouc :

On utilise le composé 1,3-dipolaire dont la synthèse est décrite ci-dessus.

Les formulations (en pce) des compositions A et B décrites dans le tableau I. Les compositions A et B à base de SBR et de silice diffèrent en ce que la composition B contient le composé 1,3-dipolaire. La composition B est conforme à l'invention, la composition A n'est pas conforme à l'invention et est la composition témoin de la composition B.

**Tableau I**

| Composition | A témoin | B conforme |
|---|---|---|
| SBR (1) | 100 | 100 |
| Composé 1,3-dipolaire (2) | - | 1.65 |
| Noir de carbone N234 | 3 | 3 |
| Silice (3) | 110 | 110 |
| Silane (4) | 9 | 9 |
| Antioxydant (5) | 2 | 2 |
| Plastifiant (6) | 58 | 58 |
| Cire anti-ozone | 1 | 1 |
| DPG (7) | 2 | 2 |
| ZnO | 1.2 | 1.2 |
| Acide stéarique | 2 | 2 |
| Sulfénamide (8) | 2 | 2 |
| Soufre | 1.5 | 1.5 |

| | | |
|---|---|---|
| (1) SBR : SBR avec 27% de motif styrène et 24% de motif 1,2 de la partie butadiénique (2) Composé 1,3-dipolaire dont la synthèse est décrite ci-dessus dans le paragraphe II.2 (3) Silice « Zeosil 1165 MP » de la société Rhodia (type HDS) (4) TESPT (« Si69 » de la société Degussa) (5) N-(1,3-diméthylbutyl)-N'-phényl-p-phénylènediamine, de la société Flexsys (6) Mélange d'huile de tournesol oléique et d'une résine C5/C9 (7) Diphénylguanidine « Perkacit » de la société Flexsys (8) N-cyclohexyl-2-benzothiazol-sulfénamide (« Santocure CBS » de la société Flexsys) | | |

On procède pour la fabrication de ces compositions de la manière suivante : on introduit dans un mélangeur interne (taux de remplissage final : environ 70% en volume), dont la température initiale de cuve est d'environ 110°C, l'élastomère, le cas échéant le composé 1,3-dipolaire qui est malaxé seul avec l'élastomère pendant environ 1 minute à 110°C, puis la silice, l'agent de couplage, ainsi que les divers autres ingrédients à l'exception du système de vulcanisation. On conduit alors un travail thermomécanique (phase non-productive) en une étape, qui dure environ 5 min à 6 minutes, jusqu'à atteindre une température maximale de « tombée » de 160°C. On récupère le mélange ainsi obtenu, on le refroidit puis on incorpore du soufre et un accélérateur type sulfénamide sur un mélangeur (homo-finisseur) à 25°C, en mélangeant le tout (phase productive) pendant un temps approprié (par exemple entre 5 et 12 min).

Les compositions ainsi obtenues sont ensuite calandrées, soit sous forme de plaques (d'une épaisseur allant de 2 à 3 mm) ou fines feuilles de caoutchouc, pour la mesure de leurs propriétés physiques ou mécaniques, soit sous la forme de profilés directement utilisables, après découpage et/ou assemblage aux dimensions souhaitées, par exemple comme produits semi-finis pour pneumatiques, en particulier pour des bandes de roulement. La réticulation est effectuée à 150°C.

### 11.4-Essais de caractérisation - Résultats :

Les résultats sont consignés dans le tableau (II) ci-après.

**Tableau (II)**

| **Composition** | A | B |
|---|---|---|
| **Propriétés à cuit** | | |
| MSA100 à 23°C | 100 | 109 |
| MSA300 à 23°C | 100 | 114 |
| MSA300/MSA100 à 23°C | 100 | 104 |
| G* à 23°C | 100 | 96 |
| tanδ max à 23°C | 100 | 89 |
| ΔG^{∗}à 23°C | 100 | 65 |

Comparativement à la composition témoin A, la composition B conforme à l'invention présente à la fois un indice de renforcement MSA300/MSA100 supérieur, un tanδ max et un ΔG^{∗} très inférieurs alors que le module G* perd seulement 4 points. La composition B présente un meilleur compromis entre la rigidité, la cohésion et l'hystérèse.

## Revendications

1. Composition de caoutchouc à base d'au moins un élastomère diénique, une charge inorganique renforçante et un composé 1,3-dipolaire choisi dans le groupe constitué par les oxydes de nitrile, les imines de nitrile et les nitrones, **caractérisée en ce que** le composé 1,3-dipolaire répond à la formule (I) :
Q-A-B (1)
Q contenant un motif -C≡N→O, -C≡N→N- ou -C=N(->O)
A, de préférence divalent, est un atome ou un groupe d'atomes reliant Q à B,
B représente une fonction ester d'acide carboxylique répondant à la formule (II)
-C(OR)=O (II)
dans laquelle R est un groupe carboné pouvant contenir au moins un hétéroatome.

2. Composition de caoutchouc selon la revendication 1 dans laquelle le groupe carboné contient 1 à 20 atomes de carbone.

3. Composition de caoutchouc selon la revendication 1 ou 2 dans laquelle A est un groupe contenant jusqu'à 20 atomes de carbone et pouvant contenir au moins un hétéroatome.

4. Composition de caoutchouc selon l'une quelconque des revendications 1 à 3 dans laquelle A est un groupe alkylène contenant de préférence 1 à 3 atomes de carbone.

5. Composition de caoutchouc selon l'une quelconque des revendications 1 à 4 dans laquelle Q comporte le motif répondant à la formule (III) : 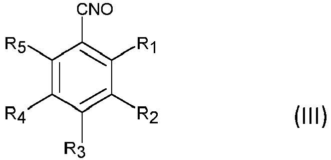 dans laquelle :
le symbole CNO représente le motif -C≡N→O,
quatre des cinq symboles R1 à R5 identiques ou différents, sont chacun un atome ou un groupe d'atomes, préférentiellement un groupe aliphatique ou un groupe aromatique et le cinquième symbole désigne un rattachement direct ou indirect à A, sachant que R1 et R5 sont tous les deux différents de H.

6. Composition de caoutchouc selon la revendication 5 dans laquelle R1, R3 et R5 sont chacun un groupe alkyle de 1 à 6 atomes de carbone.

7. Composition de caoutchouc selon la revendication 5 ou 6 dans laquelle R1, R3 et R5 sont chacun un méthyle ou éthyle.

8. Composition de caoutchouc selon l'une quelconque des revendications 5 à 7 dans laquelle le composé 1,3-dipolaire est de formule (IIIa)

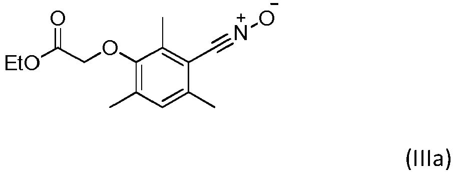

9. Composition de caoutchouc selon l'une quelconque des revendications 1 à 4 dans laquelle Q comporte le motif répondant à la formule (IV) ou (V) : 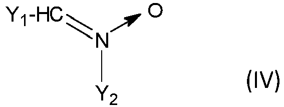 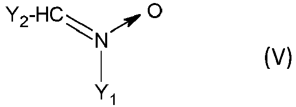 dans laquelle :
Y₁ est un groupe aliphatique, préférentiellement un groupe alkyle contenant de préférence 1 à 12 atomes de carbone, ou un groupe aromatique contenant 6 à 20 atomes de carbone, préférentiellement un groupe alkylaryle, plus préférentiellement un groupe phényle ou tolyle,
et Y₂ est un groupe aliphatique, préférentiellement un groupe hydrocarboné saturé contenant de préférence 1 à 12 atomes de carbone et comportant un rattachement direct à A, ou un groupe aromatique contenant préférentiellement 6 à 20 atomes de carbone et comportant sur son noyau benzénique un rattachement direct à A.

10. Composition de caoutchouc selon l'une quelconque des revendications 1 à 9 dans laquelle la quantité de composé 1,3-dipolaire est comprise entre 0 et 5 équivalents molaires de fonction ester d'acide carboxylique pour 100 moles d'unités monomères constituant l'élastomère diénique.

11. Composition de caoutchouc selon l'une quelconque des revendications 1 à 10 dans laquelle l'élastomère diénique est un élastomère essentiellement insaturé choisi dans le groupe constitué par les polybutadiènes, les polyisoprènes, les copolymères de butadiène, les copolymères d'isoprène et les mélanges de ces élastomères.

12. Composition de caoutchouc selon l'une quelconque des revendications 1 à 11 dans laquelle la charge inorganique est une silice.

13. Composition de caoutchouc selon l'une quelconque des revendications 1 à 12 dans laquelle la charge inorganique renforçante représente plus de 50% en masse de la masse de la charge renforçante présente dans la composition de caoutchouc.

14. Composition de caoutchouc selon l'une quelconque des revendications 1 à 13 dans laquelle la composition de caoutchouc comprend un agent de couplage silane pour lier la charge inorganique à l'élastomère diénique.

15. Composition de caoutchouc selon l'une quelconque des revendications 1 à 14 dans laquelle elle comprend un système de réticulation, préférentiellement à base de soufre.

16. Bande de roulement pour pneumatique qui comprend une composition de caoutchouc selon l'une quelconque des revendications 1 à 15.

17. Pneumatique qui comprend une composition de caoutchouc selon l'une quelconque des revendications 1 à 16, préférentiellement dans sa bande de roulement.

## Patentansprüche

1. Kautschukzusammensetzung auf Basis von mindestens einem Dienelastomer, einem verstärkenden anorganischen Füllstoff und einer 1,3-dipolaren Verbindung, die aus der Gruppe bestehend aus Nitriloxiden, Nitriliminen und Nitronen ausgewählt ist, **dadurch gekennzeichnet, dass** die 1,3-dipolare Verbindung der Formel (I) entspricht:
Q-A-B (I),
wobei
Q eine -C≡N→O-, -C≡N→N- oder -C=N(→O) -Einheit enthält,
A, das vorzugsweise zweiwertig ist, für ein Atom oder eine Gruppe von Atomen, das bzw. die Q mit B verbindet, steht,
B für eine Carbonsäureesterfunktion steht, die der Formel (II) entspricht:
-C(OR)=O (II),
in der R für eine kohlenstoffhaltige Gruppe, die mindestens ein Heteroatom enthalten kann, steht.

2. Kautschukzusammensetzung nach Anspruch 1, wobei die kohlenstoffhaltige Gruppe 1 bis 20 Kohlenstoffatome enthält.

3. Kautschukzusammensetzung nach Anspruch 1 oder 2, wobei A für eine Gruppe, die bis zu 20 Kohlenstoffatome enthält und mindestens ein Heteroatom enthalten kann, steht.

4. Kautschukzusammensetzung nach einem der Ansprüche 1 bis 3, wobei A für eine Alkylengruppe steht, die vorzugsweise 1 bis 3 Kohlenstoffatome enthält.

5. Kautschukzusammensetzung nach einem der Ansprüche 1 bis 4, wobei Q die der Formel (III) entsprechende Einheit umfasst: 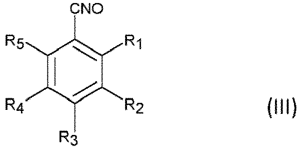 in der:
das Symbol CNO für die -C≡N→O- Einheit steht,
vier der fünf Symbole R1 bis R5 gleich oder verschieden sind und jeweils für ein Atom oder eine Gruppe von Atomen, vorzugsweise eine aliphatische Gruppe oder eine aromatische Gruppe, stehen und das fünfte Symbol eine direkte oder indirekte Bindung an A bezeichnet, mit der Maßgabe, dass R1 und R5 beide von H verschieden sind.

6. Kautschukzusammensetzung nach Anspruch 5, wobei R1, R3 und R5 jeweils für eine Alkylgruppe mit 1 bis 6 Kohlenstoffatomen stehen.

7. Kautschukzusammensetzung nach Anspruch 5 oder 6, wobei R1, R3 und R5 jeweils für ein Methyl oder Ethyl stehen.

8. Kautschukzusammensetzung nach einem der Ansprüche 5 bis 7, wobei die 1,3-dipolare Verbindung die Formel (IIIa) aufweist:

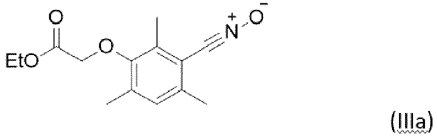

9. Kautschukzusammensetzung nach einem der Ansprüche 1 bis 4, wobei Q die der Formel (IV) oder (V) entsprechende Einheit umfasst: 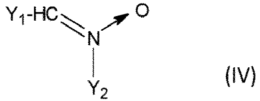 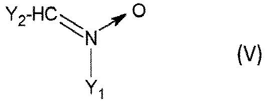 in der:
Y₁ für eine aliphatische Gruppe, vorzugsweise eine Alkylgruppe mit vorzugsweise 1 bis 12 Kohlenstoffatomen, oder eine aromatische Gruppe mit 6 bis 20 Kohlenstoffatomen, vorzugsweise eine Alkylarylgruppe, weiter bevorzugt eine Phenyl- oder Tolyl Gruppe, steht
und Y₂ für eine aliphatische Gruppe, vorzugsweise eine gesättigte Kohlenwasserstoffgruppe mit vorzugsweise 1 bis 12 Kohlenstoffatomen und einer direkten Bindung an A, oder eine aromatische Gruppe mit vorzugsweise 6 bis 20 Kohlenstoffatomen und einer direkten Bindung an A an ihrem Benzolkern steht.

10. Kautschukzusammensetzung nach einem der Ansprüche 1 bis 9, wobei die Menge der 1,3-dipolaren Verbindung zwischen 0 und 5 Moläquivalenten Carbonsäureesterfunktion pro 100 Mol Monomereinheiten, aus denen das Dienelastomer aufgebaut ist, liegt.

11. Kautschukzusammensetzung nach einem der Ansprüche 1 bis 10, wobei es sich bei dem Dienelastomer um ein im Wesentlichen ungesättigtes Elastomer aus der Gruppe bestehend aus Polybutadienen, Polyisoprenen, Butadien-Copolymeren, Isopren-Copolymeren und Mischungen dieser Elastomere handelt.

12. Kautschukzusammensetzung nach einem der Ansprüche 1 bis 11, wobei es sich bei dem anorganischen Füllstoff um eine Kieselsäure handelt.

13. Kautschukzusammensetzung nach einem der Ansprüche 1 bis 12, wobei der verstärkende anorganische Füllstoff mehr als 50 Massen-% der Masse des verstärkenden Füllstoffs in der Kautschukzusammensetzung ausmacht.

14. Kautschukzusammensetzung nach einem der Ansprüche 1 bis 13, wobei die Kautschukzusammensetzung ein Silankupplungsmittel zum Binden des anorganischen Füllstoffs an das Dienelastomer umfasst.

15. Kautschukzusammensetzung nach einem der Ansprüche 1 bis 14, wobei sie ein Vernetzungssystem, vorzugsweise auf Basis von Schwefel, umfasst.

16. Lauffläche für Reifen, die eine Kautschukzusammensetzung nach einem der Ansprüche 1 bis 15 umfasst.

17. Reifen, der eine Kautschukzusammensetzung nach einem der Ansprüche 1 bis 16 umfasst, vorzugsweise in seiner Lauffläche.

## Claims

1. Rubber composition based on at least one diene elastomer, a reinforcing inorganic filler and a 1,3-dipolar compound selected from the group consisting of nitrile oxides, nitrile imines and nitrones, **characterized in that** the 1,3-dipolar compound corresponds to formula (I):
Q-A-B (I)
Q containing a motif -C≡N→O, -C≡N→N- or -C=N(→O),
A, preferably divalent, is an atom or group of atoms linking Q to B,
B represents a carboxylic ester function corresponding to formula (II)
-C(OR)=O (II)
wherein R is a carbon-containing group that may contain at least one heteroatom.

2. Rubber composition according to Claim 1, wherein the carbon-containing group contains 1 to 20 carbon atoms.

3. Rubber composition according to Claim 1 or 2, wherein A is a group that contains up to 20 carbon atoms and may contain at least one heteroatom.

4. Rubber composition according to any one of Claims 1 to 3, wherein A is an alkylene group containing preferably 1 to 3 carbon atoms.

5. Rubber composition according to any one of Claims 1 to 4, wherein Q includes the motif corresponding to formula (III): 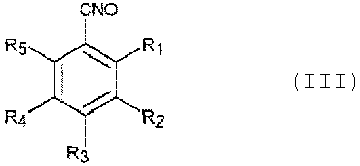 wherein:
the symbol CNO represents the motif -C≡N→O,
four of the five symbols R1 to R5, which are identical or different, are each an atom or group of atoms, preferably an aliphatic group or an aromatic group, and the fifth symbol denotes a direct or indirect point of attachment to A, where R1 and R5 are both different to H.

6. Rubber composition according to Claim 5, wherein R1, R3 and R5 are each an alkyl group having 1 to 6 carbon atoms.

7. Rubber composition according to Claim 5 or 6, wherein R1, R3 and R5 are each a methyl or ethyl group.

8. Rubber composition according to any one of Claims 5 to 7, wherein the 1,3-dipolar compound is of the formula (IIIa)

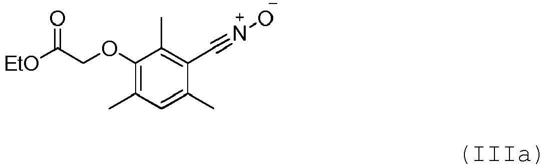

9. Rubber composition according to any one of Claims 1 to 4, wherein Q includes the motif corresponding to formula (IV) or (V): 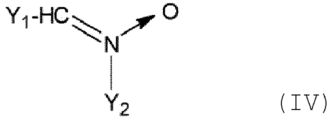 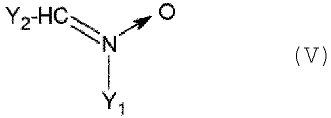 wherein:
Y₁ is an aliphatic group, preferably an alkyl group containing preferably 1 to 12 carbon atoms, or an aromatic group containing 6 to 20 carbon atoms, preferably an alkylaryl group, more preferably a phenyl or tolyl group,
and Y₂ is an aliphatic group, preferably a saturated hydrocarbyl group containing preferably 1 to 12 carbon atoms and including a direct point of attachment to A, or an aromatic group containing preferably 6 to 20 carbon atoms and including on its benzene ring a direct point of attachment to A.

10. Rubber composition according to any one of Claims 1 to 9, wherein the amount of the 1,3-dipolar compound is between 0 and 5 molar equivalents of carboxylic ester function per 100 moles of the monomeric units forming the diene elastomer.

11. Rubber composition according to any one of Claims 1 to 10, wherein the diene elastomer is a substantially unsaturated elastomer selected from the group consisting of polybutadienes, polyisoprenes, butadiene copolymers, isoprene copolymers and mixtures of these elastomers.

12. Rubber composition according to any one of Claims 1 to 11, wherein the inorganic filler is a silica.

13. Rubber composition according to any one of Claims 1 to 12, wherein the reinforcing inorganic filler represents more than 50% by mass of the mass of the reinforcing filler present in the rubber composition.

14. Rubber composition according to any one of Claims 1 to 13, wherein the rubber composition comprises a silane coupling agent for linking the inorganic filler to the diene elastomer.

15. Rubber composition according to any one of Claims 1 to 14, wherein it comprises a crosslinking system, preferably sulfur-based.

16. Tyre tread that comprises a rubber composition according to any one of Claims 1 to 15.

17. Tyre that comprises a rubber composition according to any one of Claims 1 to 16, preferably in the tread thereof.
